Europäisches Patentamt

European Patent Office

Office européen des brevets

(19)

(11) Numéro de publication: **0 078 209 B1**

(12) **FASCICULE DE BREVET EUROPEEN**

(45) Date de publication du fascicule du brevet:
06.02.85

(51) Int. Cl.⁴: **A 61 K 31/505** // (A61K31/505, 31/165)

(21) Numéro de dépôt: 82401942.6

(22) Date de dépôt: **22.10.82**

(54) **Nouvelles compositions pharmaceutiques à action antalgique.**

(30) Priorité: **23.10.81 FR 8119891**

(43) Date de publication de la demande:
**04.05.83 Bulletin 83/18**

(45) Mention de la délivrance du brevet:
**06.02.85 Bulletin 85/6**

(84) Etats contractants désignés:
**AT BE CH DE FR GB IT LI LU NL SE**

(56) Documents cités:
**FR - M - 6 805**

**DICTIONNAIRE VIDAL, 1979, Edition 55, O.V.P., page 62, Paris (FR); "Algopriv à la noramidopyrine".**

(73) Titulaire: **Laboratoires BOUCHARD, 6, rue Anna-Jacquin, F-92102 Boulogne Sur Seine (FR)**

(72) Inventeur: **Buzas, André, 6 Rue Anna Jacquin, F-92102 Boulogne (FR)**
Inventeur: **Moczar, Magdolna, 6, Rue Anna Jacquin, F-92102 Boulogne (FR)**
Inventeur: **Pierre, René, 6, Rue Anna Jacquin, F-92102 Boulogne (FR)**

(74) Mandataire: **Burtin, Jean-François, 5 Bis, rue Parmentier, F-92200 Neuilly S/Seine (FR)**

## Description

La présente invention se rapporte à de nouvelles compositions pharmaceutiques à action antalgique intense et prolongée.

Elle se rapporte, plus particulièrement, à des compositions pharmaceutiques, caractérisées en ce qu'elles renferment une association de deux principes actifs à action analgésique produisant une synergie.

Elle se rapporte spécifiquement à des compositions pharmaceutiques, caractérisées en ce qu'elles renferment une association synergique d'éthoxybenzamide et de camphosulfonate de diproqualone.

Le camphosulfonate de diproqualone est un médicament déjà connu, notamment par le brevet spécial de médicament N° 68.05M et par le brevet français N° 2068442. Il se caractérise par une toxicité faible (DL$_{50}$ chez la souris par voie sous-cutanée égale à 2,25 g/kg), et une activité antalgique sur le test de l'acide acétique (test de Koster) modérée, quelle que soit la dose (voir brevet français N° 2068442).

L'éthoxybenzamide est un médicament antalgique connu depuis la fin du siècle dernier, dont la pharmacologie détaillée n'a fait l'objet d'études que depuis 30 ans. La publication de M. Carron et coll., «Thérapie» (1952) 7, 27, a mis en évidence le fait que ce produit se caractérise par une dose minimale active relativement élevée (200 mg/kg), double de celle de l'acide acétylsalicylique, et une dose létale moyenne de 700 mg/kg chez la souris après administration orale.

L'o-éthoxybenzamide est donc considéré comme un analgésique faible doué d'une marge thérapeutique limitée.

L'invention réside donc dans la constatation surprenante que l'association de ces deux agents antalgiques faibles conduit à un effet intense et surtout prolongé. On constate également, d'une manière surprenante, une diminution de la toxicité de l'association par rapport à celle de chacun des constituants du mélange. Alors que la DL$_{50}$ chez la souris du camphosulfonate de diproqualone par voie orale est de 4250 mg/kg et que celle de l'o-éthoxybenzamide est de 700 mg/kg dans les mêmes conditions, on constate que le mélange manifeste une toxicité moindre que celle que le calcul théorique le laisserait supposer.

L'invention comprend donc les compositions pharmaceutiques renfermant une association synergique d'o-éthoxybenzamide et de camphosulfonate de diproqualone mélangée à un excipient ou à un véhicule inerte non toxique pharmaceutiquement acceptable.

D'une manière générale, le rapport entre la quantité d'o-éthoxybenzamide et celle de camphosulfonate de diproqualone peut varier de 2 pour 1 à 1 pour 4, mais de préférence elle varie du mélange à parties égales des deux principes actifs au mélange renfermant 2 parties d'o-éthoxybenzamide pour 1 partie de camphosulfonate de diproqualone.

Une composition optimale paraît être celle renfermant 200 mg de camphosulfonate de diproqualone pour 300 mg d'o-éthoxybenzamide.

Les compositions pharmaceutiques selon l'invention sont celles adaptées pour l'usage par voie orale ou rectale. Elles sont présentées sous forme de comprimés, de tablettes, de gélules, de dragées, de comprimés à double noyau ou à enrobage entérique, de suspensions buvables ou de suppositoires.

Les excipients les plus appropriés pour de telles compositions sont les amidons, la carboxyméthylcellulose, la méthylcellulose, le carbonate de calcium, le phosphate de magnésium, le talc et le stéarate de magnésium. Parmi les véhicules permettant de réaliser des formes liquides, on pourra citer le sirop de gomme, le sirop de sucre, les solutions salines et les solutions d'agents tensioactifs non ioniques comme les oléates de monosorbitan.

L'administration des compositions selon l'invention a lieu à raison de 2 à 4 prises par jour notamment sous forme de comprimés ou de gélules ou bien à raison de 1 ou 2 suppositoires par jour, pendant une période moyenne de 1 semaine, en fonction de l'état pathologique.

Les compositions selon l'invention trouvent un emploi comme médicament antalgique dans le traitement des traumatismes, des douleurs aiguës ou chroniques et plus particulièrement des dysménorrhées, des douleurs dentaires, des douleurs ostéo-articulaires, des névralgies, des céphalées et des douleurs d'origine grippale.

Les exemples suivants donnent une illustration de l'invention.

*Exemple I:*

*Comprimés à 0,3 g d'o-éthoxybenzamide et à 0,2 g de camphosulfonate de diproqualone*

| | |
|---|---|
| camphosulfonate de diproqualone | 0,200 g |
| o-éthoxybenzamide | 0,300 g |
| excipient q.s.p. 1 comprimé de | 0,600 g |

*Exemple II:*

*Suppositoires à 0,6 g d'o-éthoxybenzamide et à 0,4 g de camphosulfonate de diproqualone*

| | |
|---|---|
| camphosulfonate de diproqualone | 0,400 g |
| o-éthoxybenzamide | 0,600 g |
| excipient q.s.p. 1 suppositoire de | 2,50 g |

*Exemple III:*

*Gélules à 0,3 g d'o-éthoxybenzamide et à 0,2 g de camphosulfonate de diproqualone*

| | |
|---|---|
| camphosulfonate de diproqualone | 0,200 g |
| o-éthoxybenzamide | 0,300 g |
| lactose q.s. pour une gélule | |

*Exemple IV:*

*Etude pharmacologique du mélange selon l'invention*

a) *Toxicité aiguë*

La dose létale moyenne a été déterminée sur des lots de 10 souris mâles pesant entre 18 et 20 g. Les animaux reçoivent par tubage œsophagien le

mélange camphosulfonate de diproqualone / o-éthoxybenzamide en suspension dans l'eau distillée, à doses croissantes.

Les animaux sont gardés en observation à température constante pendant 8 d. Les morts, s'il y en a, sont dénombrés et la dose létale moyenne est déterminée selon la méthode de Lichtfield et Wilcoxon.

C'est ainsi que la $DL_{50}$ du mélange par voie orale a été trouvée à 2026 mg/kg chez la souris.

b) *Etude de l'activité analgésique comparée* de l'o-éthoxybenzamide, du camphosulfonate de diproqualone et du mélange de ces deux principes actifs.

*Premier test*

*Etude de la prolongation de l'effet analgésique de l'o-éthoxybenzamide par le camphosulfonate de diproqualone*

L'activité analgésique a été déterminée par le test des contorsions à l'acide acétique (test de Koster), qui consiste à dénombrer les torsions des souris après injection intrapéritonéale d'acide acétique. Le nombre des crampes abdominales est compté pendant 20 min. Le test a été pratiqué 30, 60, 90, 150 et 210 min. après administration des produits par voie buccale. L'activité des produits est appréciée par la diminution du nombre des crampes chez les animaux traités par rapport à celui observé chez les témoins.

Doses administrées:

| | |
|---|---|
| o-éthoxybenzamide | 100 mg/kg |
| camphosulfonate de diproqualone | 66,6 mg/kg |
| mélange o-éthoxybenzamide | 100 mg/kg |
| camphosulfonate de diproqualone | 66,6 mg/kg |

Résultats (moyenne et écarts types sur 8 souris par lot)

| | 30 min | 60 min | 90 min | 150 min | 210 min |
|---|---|---|---|---|---|
| Témoins | 32,3± 6,99 | 26,0± 1,66 | 31,2± 4,70 | 30,1± 5,04 | 28,0± 5,64 |
| o-éthoxybenzamide | 34,2± 5,16 | 24,6± 4,79 | 16,0± 3,17 | 15,3± 5,87 | 14,8± 2,72 |
| diproqualone | 31,8± 6,99 | 8,8± 2,28 | 13,6± 4,03 | 15,3± 3,85 | 27,2± 4,94 |
| mélange selon l'invention | 29,0± 6,10 | 15,5± 3,66 | 18,0± 3,62 | 13,3± 3,21 | 9,7± 2,63 |

*Discussion*

Une analyse statistique a été appliquée:

*Analyse de variance à chaque temps:*

— 30 min après l'administration des produits, aucun effet analgésique n'est observé.

— Après 1 h, la diproqualone diminue de 70% — et extrêmement significativement ($p < 0,001$) — le nombre de crampes. L'o-éthoxybenzamide est inactif. Le mélange paraît moins actif (− 48% avec $0,01 < p < 0,005$) mais la comparaison statistique montre que cette différence n'est pas significative ($p = 17,0$).
— Après 1½ h, l'o-éthoxybenzamide, la diproqualone et le mélange ont des activités analgésiques sensiblement identiques (− 39 et 54% avec $0,01 < p < 0,05$).
— Après 3½ h, la diproqualone est inactive, l'o-éthoxybenzamide réduit significativement de 50% le nombre de crampes ($0,01 < p < 0,05$) et le mélange paraît plus actif (− 67% avec $0,001 < p < 0,01$), mais l'analyse statistique montre que cette différence n'est pas significative ($p = 0,395$).

On peut donc dire qu'après une phase de latence de 30 min, la diproqualone, après 1 h, est la première à manifester un effet analgésique fort que l'on retrouve dans l'association. Puis 1½ h après l'administration orale, les effets de l'association et de ses constituants seuls sont identiques. Après 3½ h, c'est l'o-éthoxybenzamide qui apporte à l'association son efficacité pharmacologique.

*Deuxième test*

*Etude de la potentialisation de l'effet analgésique de l'o-éthoxybenzamide (ETB) par le camphosulfonate de diproqualone (DPQ).*

L'observation a été pratiquée de la cinquième à la quinzième minute après l'injection d'acide acétique. Les lots comprennent chacun 6 souris femelles (souche Charles River CD1). Les produits ont été administrés 30 min avant l'injection d'acide acétique.

Le tableau ci-dessous rassemble les résultats obtenus avec les pourcentages de diminution du nombre de crampes variables selon les doses de chacun des constituants chimiques.

| ETB (mg/kg) \ DPQ (mg/kg) | 0 | 33,3 | 66,6 | 100 | 150 |
|---|---|---|---|---|---|
| 0 | 0 | − 28 | − 7 | − 35 | − 31 |
| 50 | − 15 | − 25 | − 24 | − 51 | − 41 |
| 100 | − 53 | − 41 | − 58 | − 70 | − 93 |
| 150 | − 73 | − 48 | − 69 | − 79 | − 80 |

L'analyse statistique a été faite en prenant en compte le nombre de contractions et en regroupant tous les témoins pour augmenter la puissance du test par le grand nombre de mesures et estimer une variance résiduelle vraie; en effet, le gain de puissance compense, en moyenne, la perte de puissance due à l'intégration des variations individuelles dans la variation totale.

## Activité du camphosulfonate de diproqualone seul

Le camphosulfonate de diproqualone a une activité analgésique modérée, sans relation dose/effet significative.

## Activité de l'o-éthoxybenzamide

L'éthoxybenzamide a une activité analgésique moyenne sur ce test: sa $DE_{50}$ (dose diminuant de 50% le nombre de crampes moyen chez les témoins) est de:

$$99,6 \ (80,1 - 130,4) \ mg/kg$$

Il y a une relation dose/effet répondant à la formule:

$$y = -20,5 \ lnx + 107,9$$

dans laquelle y = nombre de crampes

x = dose en mg/kg

L'hypothèse de linéarité n'est pas contredite. La régression est significative au seuil de 1,1 pour 10 000.

## Activité du mélange o-éthoxybenzamide/camphosulfonate de diproqualone à 33,3 mg/kg

La droite de régression, à la limite de signification (risque = 5,06%), a pour formule:

$$y = -10,0 \ lnx + 62,7$$

Elle n'est pas différente de la droite éthoxybenzamide seul.

## Activité du mélange o-éthoxybenzamide/camphosulfonate de diproqualone à 66,6 mg/kg

La droite de régression, significative (risque = 1,95%) a pour formule:

$$y = -22,0 \ lnx + 115,0$$

La $DE_{50}$ de l'o-éthoxybenzamide est alors égale à:

$$9,6 \ (63,2 - 209,3) \ mg/kg$$

## Activité du mélange o-éthoxybenzamide/camphosulfonate de diproqualone à 100 mg/kg

La droite de régression, significative (risque = 2,7‰) a pour formule:

$$y = -15,1 \ lnx + 78,7$$

La $DE_{50}$ de l'o-éthoxybenzamide est alors égale à:

$$75,8 \ (39,4 - 100,1) \ mg/kg$$

La comparaison des droites éthoxybenzamide seul et (o-éthoxybenzamide/diproqualone)100 montre que les 2 droites peuvent être considérées comme parallèles et non significativement différentes (t = 1,36).

## Activité du mélange o-éthoxybenzamide/campholsulfonate de diproqualone à 150 mg/kg

La droite de régression, significative (risque = 2,4‰), a pour formule:

$$y = -6,5 \ lnx + 36,5$$

La $DE_{50}$ de l'o-éthoxybenzamide est alors égale à:

$$34,6 \ (0,2 - 60,1) \ mg/kg$$

La comparaison des droites éthoxybenzamide seul et mélange à 150 mg/kg montre qu'elles sont parallèles (t significatif à 2%).

La distance horizontale entre les 2 droites, ou puissance relative, et ses limites de confiance à 5% sont:

$$2,4 \ (1,4 - 6,2)$$

En conclusion, l'adjonction de camphosulfonate de diproqualone à l'o-éthoxybenzamide multiplie par 2,4 son activité analgésique.

**Revendications** pour les Etats contractants BE, CH, DE, FR, GB, IT, LI, LU, NL, SE

1. Compositions pharmaceutiques à action antalgique, caractérisées en ce qu'elles renferment une association synergique d'o-éthoxybenzamide et de camphosulfonate de diproqualone en mélange avec un excipient ou un véhicule inerte non toxique pharmaceutiquement acceptable.

2. Compositions pharmaceutiques selon la revendication 1, dans lesquelles l'excipient ou le véhicule inerte sont ceux adaptés pour l'usage par voie orale ou rectale.

3. Compositions pharmaceutiques selon la revendication 1, dans lesquelles le rapport entre les quantités de principes actifs varie de 2 parties d'o-éthoxybenzamide pour 1 partie de camphosulfonate de diproqualone à 1 partie d'o-éthoxybenzamide pour 4 parties de camphosulfonate de diproqualone.

4. Compositions pharmaceutiques selon la revendication 1, renfermant 300 mg d'o-éthoxybenzamide et 200 mg de camphosulfonate de diproqualone par prise unitaire pour la voie buccale.

5. Compositions pharmaceutiques selon la revendication 1, renfermant 400 mg de camphosulfonate de diproqualone et 600 mg d'o-éthoxybenzamide par prise unitaire pour la voie rectale.

**Revendications** pour l'Etat contractant: AT

1. Un procédé d'obtention de compositions phamaceutiques à action antalgique, caractérisé en ce qu'on incorpore à une association synergique d'o-éthoxybenzamide et de camphosulfonate de diproqualone un ou des exipients ou véhicules inertes, non toxiques, pharmaceutiquement acceptables, et en ce qu'on répartit le mélange ainsi formé en la forme unitaire désirée.

2. Un procédé selon la revendication 1, dans lequel l'excipient ou le véhicule inerte sont un de ceux adaptés pour l'usage par voie orale ou rectale.

3. Un procédé selon la revendication 1, dans lequel l'association synergique d'o-éthoxybenzamide et de camphosulfonate de diproqualone comprend 300 mg d'o-éthoxybenzamide et 200 mg de camphosulfonate de diproqualone en mélange avec un excipient inerte adapté pour l'usage par la voie buccale.

4. Un procédé selon la revendication 1, dans lequel l'association synergique des deux principes actifs comprend 400 mg de camphosulfonate de

7 0 078 209 8

diproqualone et 600 mg d'o-éthoxybenzamide en mélange avec un véhicule inerte adapté pour l'usage par la voie rectale.

**Patentansprüche** für die Vertragsstaaten BE, CH, DE, FR, GB, IT, LI, LU, NL, SE

1. Pharmazeutische Zubereitungen mit analgetischer Wirkung, dadurch gekennzeichnet, dass sie eine synergistische Mischung von o-Ethoxybenzamid und Diproqualonkampfersulfonat — mit einem inerten nichttoxischen Hilfsmittel oder Vehikel gemischt — enthalten.

2. Pharmazeutische Zubereitungen gemäss Anspruch 1, in denen das inerte Hilfsmittel oder Vehikel für die Verabreichung durch oralen oder rektalen Weg geeignet ist.

3. Pharmazeutische Zubereitung gemäss Anspruch 1, in denen die Beziehung zwischen den Mengen von Wirkstoffen von 2 Teilen von o-Ethoxybenzamid für 1 Teil Diproqualonkampfersulfonat bis 1 Teil o-Ethoxybenzamid für 4 Teile Diproqualonkampfersulfonat abwechselt.

4. Pharmazeutische Zubereitungen gemäss Anspruch 1, die 300 mg o-Ethoxybenzamid und 200 mg Diproqualonkampfersulfonat per Einzeleinheit, die für orale Verabreichung geeignet ist, enthalten.

5. Pharmazeutische Zubereitungen gemäss Anspruch 1, die 400 mg Diproqualonkampfersulfonat und 600 mg o-Ethoxybenzamid per Einzeleinheit, die für rektale Verabreichung geeignet ist, enthalten.

**Patentansprüche** für den Vertragsstaat: AT

1. Verfahren zur Herstellung von pharmazeutischen Präparaten mit analgetischer Wirkung, dadurch gekennzeichnet, dass einer synergistischen Kombination von o-Ethoxybenzamid und Diproqualonkampfersulfonat eine Grundmasse oder Grundmassen oder Träger zugesetzt werden, die inert, ungiftig und pharmazeutisch zulässig sind, und dass die so gewonnene Mischung in die Form der gewünschten Einheiten gebracht wird.

2. Verfahren nach Anspruch 1, dadurch gekennzeichnet, dass die inerte Grundmasse oder der inerte Träger für die orale oder rektale Verabreichung geeignet ist.

3. Verfahren nach Anspruch 1, dadurch gekennzeichnet, dass die synergistische Kombination von o-Ethoxybenzamid und Diproqualonkampfersulfonat 300 mg o-Ethoxybenzamid und 200 mg Diproqualonkampfersulfonat enthält, vermischt mit einer für die orale Verabreichung geeigneten inerten Grundmasse.

4. Verfahren nach Anspruch 1, dadurch gekennzeichnet, dass die synergistische Kombination der beiden Wirkstoffe 400 mg Diproqualonkampfersulfonat und 600 mg o-Ethoxybenzamid enthält, vermischt mit einem für die rektale Verabreichung geeigneten inerten Träger.

**Claims** for the Contracting States BE, CH, DE, FR, GB, IT, LI, LU, NL, SE

1. Pharmaceutical compositions endowed with analgetic action, characterized in that they contain a synergistic mixture of o-ethoxybenzamide and diproqualone camphosulphonate in admixture with an inert non-toxic pharmaceutically acceptable carrier or vehicle.

2. The pharmaceutical compositions according to Claim 1, in which the inert carrier or vehicle is one of those suitable for the use per oral or rectal way.

3. The pharmaceutical compositions according to Claim 1, in which the ratio of amounts of active ingredients ranges from 2 parts of o-ethoxybenzamide for 1 part of diproqualone camphosulphonate to 1 part o-ethoxybenzamide for 4 parts of diproqualone camphosulphonate.

4. The pharmaceutical compositions according to Claim 1, which contain 300 mg o-ethoxybenzamide and 200 mg diproqualone camphosulphonate per unit dosage intended for oral administration.

5. The pharmaceutical compositions according to Claim 1, which contain 400 mg diproqualone camphosulfonate and 600 mg o-ethoxybenzamide per unit dosage intended for rectal administration.

**Claims** for the Contracting State: AT

1. A process for producing pharmaceutical compositions having an analgetic action, characterized in that to a synergistic mixture of o-ethoxybenzamid and diproqualone camphosulphonate, one or more non-toxic, inert, pharmaceutically acceptable carrier or vehicle is added and the resulting mixture is distributed in the appropriate unit dosage.

2. A process according to Claim 1, in which the inert carrier or vehicle is one of those suitable for the use by oral or rectal ways.

3. A process according to Claim 1, in which the synergistic mixture of o-ethoxybenzamid and diproqualone camphosulphonate includes 300 mg o-ethoxybenzamid and 200 mg diproqualone in admixture with an inert carrier, suitable for administration by oral way.

4. A process according to Claim 1, in which the synergistic mixture of both ingredients includes 400 mg diproqualone camphosulphonate and 600 mg o-ethoxybenzamid in admixture with an inert vehicle suitable for administration by rectal way.